# EUROPEAN PATENT APPLICATION

(11) **EP 4 375 293 A1**
(43) Date of publication of application: **29.05.2024**
(21) Application number: 22208854.4
(22) Date of filing: 22.11.2022
(51) Int. Cl.: C07K 16/10, A61P 31/14

(54) **VHH ANTIBODIES AGAINST SARS-COV-2 OMICRON VARIANTS**

(71) Applicant: Max-Planck-Gesellschaft zur Förderung der Wissenschaften e.V., 80539 München (DE)
(72) Inventor: The designation of the inventor has not yet been filed
(74) Representative: Weickmann & Weickmann PartmbB

(57) **Abstract**

The present invention pertains in the fields of antibody technology, protein engineering, medicine, pharmacology, infection biology, virology, and medical diagnostics. More specifically, the present disclosure provides VHH antibodies that prevent cell entry of and infection by SARS-CoV-2 Omicron variants and that have been selected for potent cross-reaction and cross-neutralization between different Omicron variants, other variants of concern and the original Wuhan strain.

## Description

### Field of the invention

The present invention pertains in the fields of antibody technology, protein engineering, medicine, pharmacology, infection biology, virology, and medical diagnostics. More specifically, the present disclosure provides VHH antibodies that prevent cell entry of and infection by SARS-CoV-2 and that have been selected for potent neutralization of SARS-CoV-2 variants of concern, in particular Omicron variants.

### Background of the invention

The COVID-19 pandemics has plunged the world into a nearly unprecedented medical and economic crisis - mainly because only insufficient means for treatment are available. COVID-19 is caused by SARS-CoV-2 - a recently evolved virus belonging to the *Coronaviridae.* The virus infects the upper airways initially but can also cause pneumonia and more systemic infections with damages to lungs, kidneys and/or the circulatory system. Approximately 2% of the WHO-documented infections had a lethal outcome so far, whereby the probability of a severe course increases with the age of the individuals and with risk factors such as obesity, diabetes, and chronic lung or kidney diseases.

SARS-CoV-2 enters cells with the help of its homotrimeric spike protein, which docks to the ACE2 receptor of the host and subsequently mediates fusion between the viral and host cell membranes to allow entry of the viral genomic RNA into the now infected host cell. The host cell is then re-programmed to produce viral proteins, to replicate the viral RNA, and package replicated RNA into the next generation of viral particles, which are then shed in large numbers off the host cell to infect further cells.

The adaptive immune system can control viral infections by a T-cell mediated cytotoxic response that eliminates infected cells and/ or by B-cells producing antibodies that bind to the virus's surface and block infection (for a textbook view see: Murphy and Weaver, 2016). Such infection-blocking antibodies are referred to as neutralizing ones. Typically, however, only a small fraction of produced anti-viral antibodies also has neutralizing properties.

Immunoglobulin G (IgG) is not only the most abundant class of antibodies but also the most important one in terms of long-term immunity. IgGs are built from four polypeptide chains, two light and two heavy ones. They contain two antigen-binding sites formed each by one heavy and one light chain. Such a composite binding site can confer a virus-neutralizing activity. In addition, IgGs comprise an Fc fragment, formed only by the two heavy chains. The Fc fragment is a prototypic immune effector domain that can interact with cell surface receptors (called Fc receptors) as well as with components of the complement system.

In some cases, however, antibodies might even help viral entry by an effect called ADE (for antibody-dependent enhancement) that is mediated by Fc-receptor interactions (Kliks *et al.*, 1989; Littaua *et al.*, 1990). Likewise, the immune system might aggravate the condition of patients with COVID-19 by a systemic over-reaction that manifests itself as a cytokine storm (Chen *et al.,* 2020; Mehta *et al.,* 2020) or an inappropriate complement activation (Magro *et al.,* 2020).

An effective immune response will either prevent a virus from establishing an infection in the first place or reduce, after an initial infection, the virus-load to non-pathogenic levels. An insufficiently controlled SARS-CoV-2 infection, however, might have a lethal outcome. In survivors, it often causes permanent damage (e.g., lung dysfunction) and impairs quality of life. An efficient anti-viral therapy would be highly desirable, ideally with orthogonal lines of treatment that target distinct steps of the viral infection cycle independently.

Remdesivir represents so far one line of treatment. This nucleoside analogue inhibits the SARS-CoV-2 RNA polymerase. As of May 2020, clinical studies gave mixed outcomes: from no decrease in mortality to a modest reduction in hospitalization time (Wang *et al.,* 2020b).

Another treatment option for severely infected patients is passive immunization with polyclonal antibodies/blood plasma from individuals who have already recovered from COVD-19 (Duan *et al.*, 2020). This approach is considered as a last resort and generally has several drawbacks, such as the risk of ADE in case of an insufficient neutralization, limited availability of sera from convalescent individuals, inevitable batch-to-batch variability of plasma samples, the risk of transmitting other infectious diseases, and all other potential adverse effects when large amounts of antibodies of mixed specificity are transferred from one person to another.

A more advanced approach is the isolation of monoclonal antibodies from SARS-CoV-2 infected or convalescent patients (Wang *et al.,* 2020a). These can be characterized in-depth and produced reproducibly in a recombinant form. This solves some of the issues raised for the plasma-therapy mentioned above. However, monoclonal antibodies are very costly to produce, making it hard to imagine that it will be feasible to scale up production to levels needed for the treatment of large numbers of patients. In addition, immunological side effects, such as aggravating cytokine storms, inappropriate complement activation, or ADE, cannot be ruled out either, mainly due to the Fc region present in human/ humanized antibodies.

By now, several SARS-CoV-2 variant strains with mutated RBDs, i.e., escape mutants, have emerged that are causing devastating outbreaks. The emergence of such escape mutants is presumably driven by the selective pressure of the immune system, particularly in immunocompromised, persistently infected patients (Clark *et al.,* 2021; Starr *et al.,* 2021). Escape mutants can bypass antibody-based immunity in previously infected or vaccinated individuals. This has the potential of sustaining infection waves in populations that have acquired herd immunity against earlier strains.

Escape mutants also pose a tremendous challenge for therapeutic antibodies, which may be rendered ineffective by a given mutation. Indeed, several monoclonal anti-SARS-CoV-2 antibodies developed for treating COVID-19 are ineffective against the South African and Brazilian variants (Garcia-Beltran *et al.,* 2021; Hoffmann *et al.,* 2021; Li *et al.,* 2021; Tada *et al.,* 2021; Wibmer *et al.,* 2021; Zhou *et al.,* 2021).

Camelids are unusual in that they produce heavy chain-only antibodies, whose simpler antigen-binding domain can easily be cloned and recombinantly expressed in bacteria or yeast (Arbabi Ghahroudi *et al.,* 1997). Such isolated antigen-binding domains are then referred to as nanobodies or VHH antibodies (variable domain of heavy homodimer antibodies). They are just ~14kDa in size - about one-tenth of a traditional IgG molecule. They have found numerous useful applications, for example as crystallization chaperones (Rasmussen *et al.*, 2011; Chug *et al.*, 2015), as tools in affinity chromatography, for localizing proteins in cells or as an animal-friendly substitute for secondary antibodies (Pleiner *et al.,* 2015, 2018).

VHH antibodies recognizing the SARS-CoV-2 spike protein S1 domain are described in co-owned application PCT/EP2021/071309 and in EP21209549.1 the content of which is herein incorporated by reference. These show good binding and neutralization characteristics with several SARS-CoV-2 variant strains.

The SARS-CoV-2 variant of concern (VOC) Omicron (B.1.1.529, now called BA.1) is an unusual variant, harboring more than 30 escape mutations in the SARS-CoV-2 Spike protein alone. The Omicron variant has later diverged giving rise to >300 subvariants, notable ones being BA.2, BA.2.75, BA.4, BA.5 and BQ.1. Unlike the earlier VOCs, the escape mutations in Omicron and subvariants thereof reside not only in epitope 1 but also in epitope 2, hampering binding ability of most antibodies, hence limiting their effectiveness in virus neutralization.

It was an object of the present invention to provide VHH antibodies showing improved binding and neutralization characteristics with SARS-CoV-2 variant strains including Omicron variants.

### Summary of the invention

According to the present invention, SARS-CoV-2-neutralizing VHH antibodies are provided, which are suitable for the effective treatment of COVID-19 patients. Specifically, the invention provides VHH antibodies that neutralize virus already at a very low concentration. Further, the invention provides stable VHH antibodies.

In particular embodiments, the VHH antibodies are monomeric VHH antibodies.

In further embodiments, the VHH antibodies are multimeric VHH antibodies, particularly homotrimeric VHH antibodies or heterodimeric VHH antibodies.

A first aspect of the invention relates to an VHH antibody recognizing the SARS-CoV-2 spike protein of the Omicron variant or a subvariant thereof, particularly the receptor-binding domain (RBD) of the SARS-CoV-2 Omicron variant BA.5, BA.1, BA.2, BA.2.75, BA.4 and/or BQ.1.

The VHH antibody is selected from the group of VHH antibodies comprising a CDR3 sequence as disclosed herein or a related CDR3 sequence. In certain embodiments, the VHH antibody is selected from the group of VHH antibodies comprising a combination of CDR1, CDR2 and CDR3 sequences as disclosed herein or a related combination of CDR1, CDR2 and CDR3 sequences. In certain embodiments, the VHH antibody is selected from the group of antibodies comprising a VHH sequence as disclosed herein.

In certain embodiments, the SARS-CoV-2-neutralizing VHH antibody is a stable, particularly a thermostable or a hyperthermostable VHH antibody.

In certain embodiments, the VHH antibody as described above is covalently or non-covalently conjugated to a heterologous moiety, which may be selected from a labeling group, a capture group or an effector group.

In certain embodiments, the VHH antibody as described above is fused to a heterologous polypeptide moiety, which may be selected from a multimerization module, e.g., dimerization, trimerization or tetramerization module. In particular embodiments, the multimerization module is trimerization module.

In certain embodiments, the VHH antibody is conjugated to one or several polymer moieties, preferably hydrophilic polymer moieties, such as polyethylene glycol (PEG), to increase the molecular weight of the antibody conjugate and, thus, delay renal clearance. The molecular weight of a polymer moiety may vary over a broad range, for example in the range of about 5 kDa to about 80 kDa. Such coupling may be performed through e.g., amino or carboxyl groups already present in the VHHs and/or through the side chains of lysine, aspartic acid, glutamic acid or cysteine residues, or through engineered backbone or side chains of other amino acids, and involve known chemistries for forming amide bonds, secondary amine bonds or thioether bonds.

In certain embodiments, the VHH antibody as described above is directed against the SARS-CoV-2 spike protein receptor-binding domain (RBD). In certain embodiments, the VHH antibody binds to the RBD of a SARS-CoV-2 variant having the immune-escape mutations G339D, S371L, S373P, S375F, K417N, N440K, , S477N, T478K, E484A, G496S, Q498R, N501Y, and/or Y505H. In certain embodiments, the VHH antibody binds to the RBD of a SARS-CoV-2 variant having also at least one of the immune-escape mutations G446S, Q493R, L452R, and F486V. In certain embodiments, the VHH antibody binds to the RBD of the SARS-CoV-2 Omicron variant BA.5, BA.1, BA.2, BA.2.75, BA.4 and/or BQ.1 or to the RBD of a SARS-CoV-2 having the immune-escape mutations G339D, S371L, S373P, S375F, K417N, N440K, G446S, S477N, T478K, E484A, Q493R, G496S, Q498R, N501Y, and/or Y505H (BA.1) or G339D, S371L, S373P, S375F, K417N, N440K, L452R, S477N, T478K, E484A, F486V, G496S, Q498R, N501Y, and/or Y505H (BA.5).

The numbering of the indicated positions in the RBD in which mutations are present refers in each case to the spike protein of the original Wuhan strain (SEQ ID NO. 31).

In certain embodiments, the VHH antibody is capable of virus neutralization. In certain embodiments, the SARS-CoV-2-neutralizing VHH antibody neutralizes a SARS-CoV2 variant, e.g., a variant comprising at least one mutation in the RBD, particularly at position G339D, S371L, S373P, S375F, K417N, N440K, G446S, L452R, S477N, T478K, E484A, F486V, Q493R, G496S, Q498R, N501Y, and/or Y505H. In certain embodiments, the VHH antibody cross-neutralizes the SARS-CoV-2 Omicron variant or a subvariant thereof, in particular BA.5, BA.1, BA.2.75, BQ.1, the Wuhan strain, and the Delta variant.

The sequences of the variable regions (CDRs) of preferred VHH antibodies of the present invention as well as assignment of SEQ ID NOs for VHHs and CDRs are listed in the following Table 1:

### Table 1

**Table 1: Anti-Omicron BA. 1/BA.5 VHHs and their CDR regions.**

| | SEQ ID NO. | CDR1 | SEQ ID NO. | CDR2 | SEQ ID NO. | CDR3 | SEQ ID NO. |
|---|---|---|---|---|---|---|---|
| Re9H03 | 19 | FTLDYYAIG | 20 | SRISSSDGSTDYADS | 21 | TVPGTYYSGNWYYTWHPKAVDY | 22 |
| Ma16B06 | 15 | SILQIWAMK | 16 | ATIPNSGEPFYASS | 17 | VNEGVPVREY | 18 |
| Ma28H02 | 11 | IGLSYYGVG | 12 | ACMHNTDGNPVYAPS | 13 | VGPLSPGITCRIPTTLGYDD | 14 |
| Ma28H11 | 7 | LTLDHYAIG | 8 | ACVSDDERSLYAPS | 9 | SGPVVPGTLCRIPTVLGYDD | 10 |
| Ma28D01 | 1 | LGLDYYQIG | 2 | SCFRSSDEIRYYADS | 3 | VAPQTYYHGTWYGECAAAGMDS | 4 |
| Ma28F04 | 5 | LGLDYYQIG | 2 | SCFRSSDEIRYYADP | 6 | VAPQTYYHGTWYGECAAAGMDS | 4 |

| | | | | | | | |
|---|---|---|---|---|---|---|---|
| *Re9H03 is described in* PCT/EP2021/071309 *and provided for comparative purposes.* | | | | | | | |

A list of complete sequences of VHH antibodies is shown at the end of the specification.

There are several methods known in the art for determining the CDR sequences of a given antibody molecule, but there is no standard unequivocal method. Determination of CDR sequences from antibody heavy chain variable regions can be made according to any method known in the art, including, but not limited to, the methods known as KABAT, Chothia, and IMGT. A selected set of CDRs may include sequences identified by more than one method, namely, some CDR sequences may be determined using KABAT and some using IMGT, for example. According to some embodiments of the present invention, the CDR sequences of the mAb variable regions are determined using the KABAT methods. CDRs may also be defined through a multiple alignment (with many other VHH antibodies), to identify the hot-spots of variability and relate them to a standard VHH antibody structure. It is also possible to define CDRs by analysing the structure of the VHH antibody and deciding what is a loop and what is the antibody's scaffold. In some cases, CDR-adjacent residues are also variable, and are therefore included in the CDR definition.

A further aspect of the present invention relates to a set of two or more different VHH antibodies, wherein the different VHH antibodies may recognize different epitopes, particularly different epitopes on the RBD, and more particularly non-overlapping epitopes on the RBD, and wherein at least one VHH antibody is as described above.

A VHH antibody described above is suitable for use in medicine, e.g., human medicine, particularly for use in therapy, e.g., in the prevention or treatment of a disorder caused by and/or associated with an infection with SARS-CoV-2, or in diagnostics, e.g., for detecting SARS-CoV-2 virus or viral components in a patient sample, e.g., in a body fluid or tissue sample, or in research.

Still a further aspect of the invention relates to a nucleic acid molecule encoding a VHH antibody as described above, particularly in operative linkage with a heterologous expression control sequence, a vector comprising said nucleic acid molecule or a recombinant cell or non-human organism transformed or transfected with said nucleic acid molecule or said vector.

Still a further aspect of the invention relates to a method for recombinant production of a VHH antibody as described above, comprising cultivating a cell or an organism as described above in a suitable medium and obtaining the VHH antibody from the cell or organism or from the medium.

Still a further aspect of the invention relates to a method for the prevention or treatment of a disorder caused by and/or associated with an infection with SARS-CoV-2 including a SARS-CoV2 mutant, comprising administering an effective dose of the VHH antibody as described above or the set of at least two different VHH antibodies as described above to a subject in need thereof, particularly to a human subject infected with SARS-CoV-2 or at risk from being infected with SARS-CoV-2.

A non-limitative list of VHH antibodies of the present invention and their utility for virus neutralization is shown in the following Table 2:

### Table 2

**Table 2: Anti-Omicron BA.1/BA.5 VHHs, their affinities for the SARS-CoV-2 RBD (expressed as KD values) and neutralization potencies.**

| **VHH** | **Thermostability** | **RBD-Affinity (pM)** | | | | | | **Lowest virus neutralizing concentration (pM; IC99)** | | | **Lowest pseudovirus neutralization concentration (nM; IC90)** | | |
|---|---|---|---|---|---|---|---|---|---|---|---|---|---|
| | | **WT** | **Delta** | **Omicron BA.1** | **BA.2.75** | **BA.5** | **BQ.1** | **WT** | **Delta** | **BA.1** | **BA.2** | **BA.2.75** | **BA.5** |
| Re5D06 | | ≤10 | 200 | n.b. | n.b. | n.b. | n.b. | | | | | | |
| Re6H06 | | ≤10 | 100 | n.b. | ≥10⁶ | n.b. | n.b. | | | | | | |
| Re9B09 | | 30 | 300 | 2000 | 2000 | 7000 | ≥10⁶ | | | | | | |
| Re32D03 | >95°C | ≤10 | ≤10 | n.b. | 350 | 20000 | 50000 | 50 | 50 | >5000 | | | |
| **Ma28H11** | **>95°C** | **≤10** | **30** | **30000** | **600** | **300** | **800** | **170** | **50** | **>5000** | **100** | **1** | **10** |
| **Ma16B06** | **57°C** | **≤10** | **n.b.** | **40** | **60** | **20000** | **25000** | **1700** | **>5000** | **50** | **1** | **1** | **>100** |
| **Ma28D01** | **>95°C** | **≤10** | **15** | **200** | **300** | **30** | **55** | **500** | | **1700** | **1** | **1** | **1** |
| **Ma28F04** | **>95°C** | **≤10** | **30** | **150** | **200** | **30** | **45** | **500** | **500** | **500** | **1** | **1** | **1** |

| | | | | | | | | | | | | | |
|---|---|---|---|---|---|---|---|---|---|---|---|---|---|
| # When no binding signal was detected, KDs were reported as n.b. for "no binding". *VHH antibodies Re5D06, Re6H06, Re9B09, Re9H03 and Re32D03 are described in* PCT/EP2021/071309 *or* EP21209549.1*, supra. All other VHH antibodies are described for the first time in the present invention.* | | | | | | | | | | | | | |

### Embodiments of the invention

In the following, specific embodiments of the invention are disclosed as follows:
1. A VHH antibody recognizing the SARS-CoV-2 spike protein S1 domain, particularly the receptor-binding domain (RBD) of the SARS-CoV-2 S1 domain comprising
   (a) a CDR3 sequence as shown in SEQ. ID NO:4, 10, 14 or 18,
   (b) a CDR3 sequence, which has an identity of at least 80%, at least 90% or at least 95% to a CDR3 sequence of (a), or
   (c) a VHH antibody, which competes with a VHH antibody of (a) for the binding to the SARS-CoV-2 spike protein S1 domain, particularly the receptor-binding domain (RBD) of the SARS-CoV-2 S1 domain.
2. The VHH antibody according to embodiment 1 comprising
   (a) a combination of CDR1, CDR2 and CDR3 sequences as shown in SEQ.ID NO:2-4 or 2, 6, and 4; 8-10; 12-14; or 16-18,
   (b) a combination of CDR1, CDR2 and CDR3 sequences which has an identity of at least 80%, at least 90% or at least 95% to a combination of CDR1, CDR2 and CDR3 sequences of (a), or
   (c) a VHH antibody, which competes with a VHH antibody of (a) for the binding to the SARS-CoV-2 spike protein S1 domain, particularly the receptor-binding domain (RBD) of the SARS-CoV-2 S1 domain.
   Particular examples of the VHH antibody according to embodiment 2 are VHH antibodies comprising a set of 3 CDRs, wherein the set is selected from:
   i. CDR1 having SEQ ID NO. 2, CDR2 comprising the sequence of SEQ ID NO. 28, and CDR3 having SEQ ID NO. 4;
   ii. CDR1 having SEQ ID NO. 8, CDR2 having SEQ ID NO. 9, and CDR3 having SEQ ID NO. 10;
   iii. CDR1 having SEQ ID NO. 12, CDR2 having SEQ ID NO. 13, and CDR3 having SEQ ID NO. 14; and
   iv. CDR1 having SEQ ID NO. 16, CDR2 having SEQ ID NO. 17, and CDR3 having SEQ ID NO. 18.
   In a particular aspect, the VHH antibody according to embodiment 2 comprises a set of 3 CDRs, wherein CDR1 is SEQ ID NO. 2, CDR2 is the sequence SCFRSSDEIRYYAD**X** (SEQ ID NO. 29), wherein X is any natural amino acid residue, and CDR3 is SEQ ID NO. 4.
   In a specific aspect, the VHH antibody comprises a set of 3 CDRs, wherein CDR1 is SEQ ID NO. 2, CDR2 is selected from SEQ ID NO: 3 and SEQ ID NO: 6, and CDR3 is SEQ ID NO. 4.
3. The VHH antibody according to embodiment 1 or 2 comprising
   (a) a VHH sequence as shown in SEQ. ID NO: 1, 5, 7, 11, or 15,
   (b) a sequence which has an identity of at least 80%, at least 90%, at least 95% or at least 99% to a VHH sequence of (a), or
   (c) a VHH antibody, which competes with a VHH antibody of (a) for the binding to the SARS-CoV-2 spike protein S1 domain, particularly the receptor-binding domain (RBD) of the SARS-CoV-2 S1 domain.
   Particular examples of the VHH antibody according to embodiment 2 are VHH antibodies comprising the sequence of 
QVQLVESGGGLVQPGGSLRLSCVVSX1LGLDYYQIGWFRQAPGKEREGVSC
FRSSDEIRYYADX2VKGRFTTFRDNAGTTVYLQMNSLKPEDTGVYYCAVAPQT
YYHGTWYGECAAAGMDSWGKGTX3VTVSS (SEQ ID NO: 30)
 wherein: X₁ is selected from aspartic acid (D) and glutamic acid (E), X₂ is selected from proline (P) and serine (S), and X₃ is selected from leucine (L) and glutamine (Q).
4. The VHH antibody of any one of embodiments 1-3, which binds to the RBD of the SARS-CoV-2 Omicron strain BA.5, BA.1, BA.2.75, and/or BQ.1 with an affinity expressed as dissociation constant KD of about 1000 pM or less, of about 500 pM or less, of about 100 pM or less or of about 50 pM or less.
5. The VHH antibody of any one of embodiments 1-4, which neutralizes SARS-CoV-2, e.g., the SARS-CoV-2 Omicron variant BA.5, BA.1, BA.2.75, and/or BQ.1.
6. The VHH antibody of embodiment 5, which neutralizes a SARS-CoV2 variant comprising a spike protein RBD including at least one mutation at position G339D, S371L, S373P, S375F, K417N, N440K, G446S, L452R, S477N, T478K, E484A, F486V, Q493R, G496S, Q498R, N501Y, and/or Y505H.
7. The VHH antibody of embodiment 5 or 6, which neutralizes a variant of concern including Omicron and subvariants thereof, in particular BA.5 and/or BA.1.
8. The VHH antibody of any one of embodiments 5-7, which neutralizes a SARS-CoV-2 Omicron variant, in particular BA.5 and/or BA.1, at a concentration of about 5 nM or less, of about 1000 pM or less, or of about 500 pM or less.
9. The VHH antibody of any one of embodiments 5-8, which cross-neutralizes the SARS-CoV-2 Omicron variant BA.5, BA.1, BA.2.75, and/or BQ.1, the Wuhan/Wildtype strain, and the Delta variant, particularly at a concentration of about 5 nM or less, of about 1000 pM or less, of about 500 pM or less, of about 170 pM or less, or of about 100 pM or less.
10. The VHH antibody of any one of embodiments 1-9, which is stable, particularly thermostable or hyperthermostable.
11. The VHH antibody of embodiment 10, which has a melting temperature of at least about 65°C, of at least about 80°C, of at least 90°C or of at least about 95°C when measured under non-reducing conditions.
12. The VHH antibody of embodiment 10 or 11, which has an aggregation temperature of at least about 50°C, of at least about 60°C, of at least 70°C or of at least about 80°C when measured under non-reducing conditions.
13. The VHH antibody of any one of embodiments 1-12, which is selected from antibody Ma28D01 comprising a VHH sequence as shown in SEQ. ID NO: 1 or a VHH antibody, which is a variant thereof.
14. The VHH antibody of any one of embodiments 1-12, which is selected from antibody Ma28F04 comprising a VHH sequence as shown in SEQ. ID NO: 5 or a VHH antibody, which is a variant thereof.
15. The VHH antibody of any one of embodiments 1-12, which is selected from antibody Ma28H11 comprising a VHH sequence as shown in SEQ. ID NO: 7 or a VHH antibody, which is a variant thereof.
16. The VHH antibody of any one of embodiments 1-12, which is selected from antibody Ma28H02 comprising a VHH sequence as shown in SEQ. ID NO: 11 or a VHH antibody, which is a variant thereof.
17. The VHH antibody of any one of embodiments 1-12, which is selected from antibody MA16B06 comprising a VHH sequence as shown in SEQ. ID NO: 15 or a VHH antibody, which is a variant thereof.
18. The VHH antibody of any one of embodiments 1-17, which is non-glycosylated.
19. The VHH antibody of any one of embodiments 1-18, which is produced in a bacterium, e.g., *E. coli,* or in a yeast, e.g., *Pichia pastoris.*
20. The VHH antibody of any one of embodiments 1-18, which is produced in a human or animal cell, e.g. a mammalian cell.
21. The VHH antibody of any one of embodiments 1-20, which is a monomeric VHH antibody.
22. The VHH antibody of any one of embodiments 1-20, which is a multimeric VHH antibody.
23. The VHH antibody of embodiment 22, which is a homo-multimeric, e.g., a homotrimeric VHH antibody comprising 3 subunits, wherein each subunit comprises a monomeric VHH antibody fused to a trimerization module, e.g., a collagen trimerization moiety, particularly a human collagen moiety, or a lung surfactant protein D moiety.
24. The VHH antibody of embodiment 22, which is a heterodimeric VHH antibody, particularly a covalently linked VHH heterodimer comprising a first VHH antibody and a second VHH antibody wherein the first VHH antibody and the second VHH antibody bind to different epitopes on the RBD of the SARS-CoV-2 S1 domain.
25. The VHH antibody of embodiment 24, which comprises two VHH antibodies that bind to non-overlapping RBD epitopes.
26. The VHH antibody of any one of embodiments 1-25, which is covalently or non-covalently conjugated to a heterologous moiety, e.g., a labeling group, a capture group or an effector group, wherein the heterologous moiety is particularly selected from a fluorescence group, biotin, an enzyme such as a peroxidase, phosphatase or luciferase, a hapten, an affinity tag, or a nucleic acid such as an oligonucleotide.
27. The VHH antibody of any one of embodiments 1-26, which is fused to a heterologous polypeptide moiety, or which is fused to an Fc Fragment, or to serum albumin or an albumin-binding moiety.
28. The VHH antibody of any of embodiments 1-27, which is conjugated to one or several polymer moieties, preferably hydrophilic polymer moieties, such as polyethylene glycol (PEG).
29. A set of two or more different VHH antibodies recognizing the SARS-CoV-2 spike protein S1 domain, particularly the receptor-binding domain (RBD) of the SARS-CoV-2 S1 domain, comprising at least one VHH antibody of any of embodiments 1-28, particularly a monomeric VHH antibody of embodiment 13 or 14.
30. The set of embodiment 29, wherein the different VHH antibodies recognize different epitopes on the RBD, particularly non-overlapping epitopes on the RBD.
31. The VHH antibody of any one of embodiments 1-28 or the set of embodiment 29 or 30 for use in medicine, e.g., human medicine, particularly for use in therapy or diagnostics.
32. The VHH antibody of any one of embodiments 1-28 or the set of embodiment 29 or 30 for use in the prevention or treatment of a disorder caused by and/or associated with an infection with SARS-CoV-2 including an infection with a SARS-CoV-2 escape mutant.
33. The VHH antibody of any one of embodiments 1-28 or the set of embodiment 29 or 30 for use in the prevention or treatment of COVID-19 in a human subject.
34. The VHH antibody of any one of embodiments 1-28 or the set of embodiment 29 or 30 for use in the detection of SARS-CoV-2 virus.
35. Use of the VHH antibody of any one of embodiments 1-28 or the set of embodiment 29 or 30 for detecting SARS-CoV-2 virus or viral components in a patient sample, e.g., in a body fluid or tissue sample.
36. Use of the VHH antibody of any one of embodiments 1-28 or the set of embodiment 29 or 30 for detecting SARS-CoV-2 virus or viral components in a virus culture, e.g., in a culture of SARS-CoV-2 or variants thereof including pseudo typed variants, or in a genetically modified organism, e.g., in an organism producing viruses or viral components.
37. The use of embodiment 36 for monitoring, quantification and/or quality control during production of viruses or viral components.
38. A nucleic acid molecule encoding a VHH antibody or a subunit of a VHH antibody according to any one of embodiments 1-28, preferably in operative linkage with a heterologous expression control sequence.
39. A vector comprising a nucleic acid molecule according to embodiment 38.
40. A recombinant cell or non-human organism transformed or transfected with a nucleic acid molecule according to embodiment 38 or a vector according to embodiment 39.
41. The cell or organism of embodiment 40, which is selected from a bacterium such as *E. coli Bacillus sp.,* a unicellular eukaryotic organism, e.g., yeast such as *Pichia pastoris,* or *Leishmania,* an insect cell, a mammalian cell or a plant cell.
42. A method for recombinant production of a VHH antibody of any one of embodiments 1-28, comprising cultivating a cell or an organism of embodiment 40 or 41 in a suitable medium and obtaining the VHH antibody from the cell or organism or from the medium.
43. The method of embodiment 42, comprising cultivating a yeast such as *Pichia pastoris* and obtaining the VHH antibody from the medium.
44. A method for the prevention or treatment of a disorder caused by and/or associated with an infection with SARS-CoV-2, comprising administering an effective dose of the VHH antibody of any one of embodiments 1-28 or the set of embodiment 29 or 30 to a subject in need thereof, particularly to a human subject.

### Detailed description of the invention

The primary aim of this aspect of the invention has been to generate, select, engineer and optimize SARS-CoV-2-neutralising VHH antibodies for effective treatment of COVID-19 patients. Specifically, we aimed for VHH antibodies that are capable of virus neutralization of SARS-CoV-2 variants, particularly SARS-CoV-2 Omicron variants and subvariants thereof, and of cross-neutralization of different SARS-CoV-2 variants already at a very low concentration. Further, we aimed for VHH antibodies that have sufficient stability for pharmaceutical applications.

According to the present invention, VHH antibodies are provided which cross-neutralize SARS-CoV-2 Omicron variants of concern at very low concentrations.

In previous work, e.g., as described in PCT/EP2021/071309, supra, VHH antibodies from three alpacas immunized with SARS-CoV-2 S1 and RBD were obtained including neutralizing VHH antibodies directed against two RBD epitopes, namely epitope 1 (the main epitope defined by VHH antibody Re5D06 shown SEQ ID NO: 1) and epitope 2 (the epitope defined by the VHH antibody Re9F06 shown in SEQ ID NO: 115). Epitope 1 has a very large overlap with ACE2, epitope 2 only a small overlap. Epitope 1-binders such as Re5D06 were found to be more potent in neutralization (neutralizing down to concentrations of 50 pM) than epitope 2 binders (so far not better than 1.7 nM). However, epitope 1 was found to be mutated in all variants of concern, namely in Alpha (N501Y), Beta (N501Y, E484K, K417N), Gamma (N501Y, E484K, K417T), Delta (L452R, T478K), Epsilon (L452R), Mu (E484K, N501Y). These escape mutations impede binding and neutralization by epitope 1-binding VHH antibodies, rendering them less suitable in therapeutic applications.

The SARS-CoV-2 variant of concern (VOC) Omicron (B.1.1.529, now called BA.1) harbours more than 30 escape mutations in the SARS-CoV-2 Spike protein alone. 15 of these, namely G339D, S371L, S373P, S375F, K417N, N440K, G446S, S477N, T478K, E484A, Q493R, G496S, Q498R, N501Y, Y505H, are in the RBD domain. Unlike the earlier VOCs, these escape mutations reside not only in epitope 1 but also in epitope 2, hampering binding ability of most of the previous VHH antibodies, hence limiting their effectiveness in virus neutralization.

To overcome this problem, we performed additional phage display screen using the alpaca-derived libraries that gave rise to VHH antibodies described in EP21209549.1 (e.g., Re32D03 and Re30H02 classes) with Omicron BA.1 RBD as a bait. We obtained completely new VHH antibodies, which did not include any of the previously isolated classes or their variants. These VHH antibodies, however, failed to neutralize SARS-CoV-2 Omicron BA.1, although most of them had very high affinity to Omicron BA.1 RBD. Despite our efforts, we were unable to obtain potently neutralizing VHH antibodies from these libraries.

It was well possible that the vast number of Omicron escape mutations, especially those at the epitopes 1 and 2, interfered with VHH antibodies -that would normally bind to these neutralizing epitopes- leaving only non-neutralizing ones. Accordingly, we re-immunized the same three alpacas with Omicron BA.1 Spike and RBD proteins, prepared immune libraries, and performed phage display screening using Omicron BA.1 RBD as a bait. This strategy yielded one new VHH class, namely Ma16B06 (Tables 1 and 2).

The Omicron variant has later diverged giving rise to >300 subvariants, notable ones being BA.2, BA.2.75, BA.4, BA.5 and BQ.1. Omicron BA.5 remains the predominant VOC, constituting >60% of the new infections in USA (as of October 2022). Yet, BA.5 has only four epitope 1 substitutions with respect to Omicron BA.1, two reversal mutations namely S446G and R493Q; and two further escape mutations namely L452R and F486V.

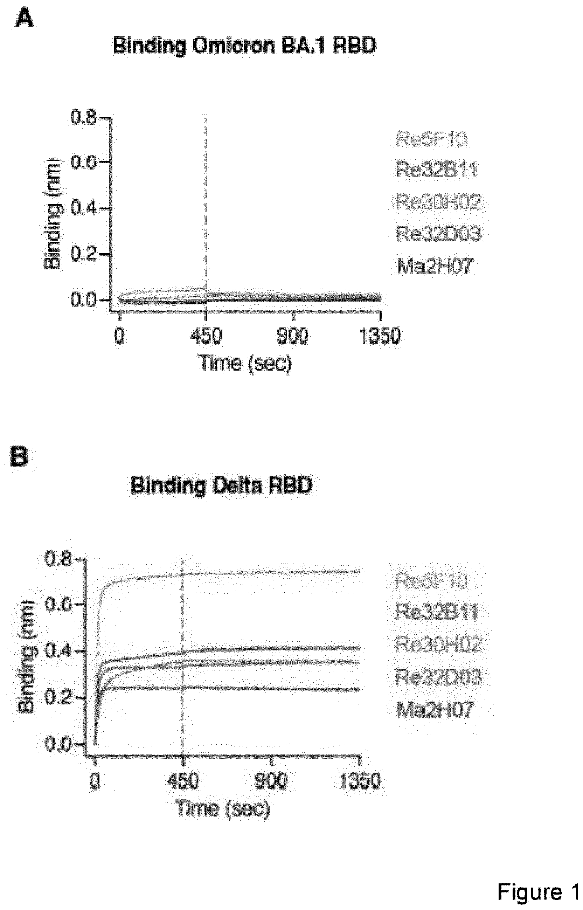

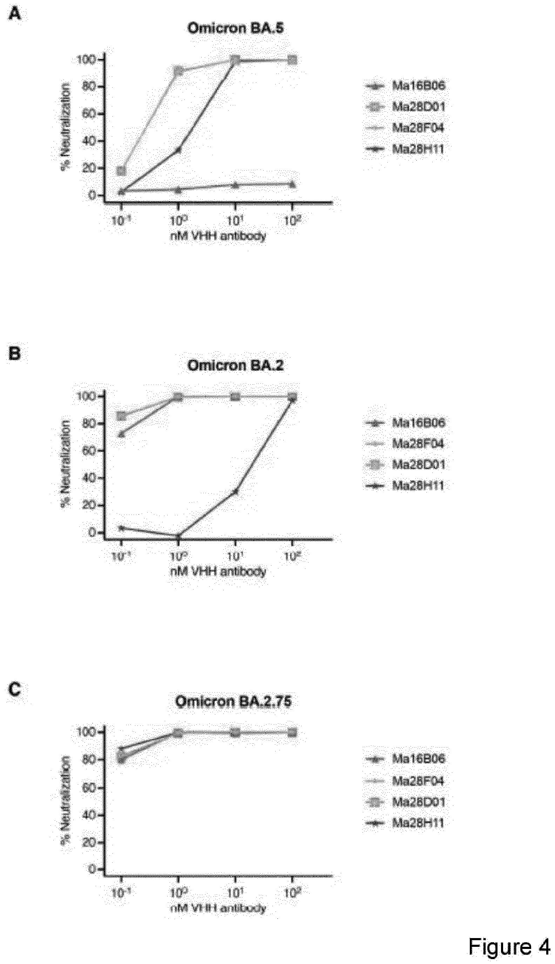

These mutations impaired VHH Ma16B06 binding to Omicron BA.5 RBD (Figure 1) and hence diminished Omicron BA.5 neutralization potency (Figure 4). Subsequently, we re-immunized the alpacas with Omicron BA.5 as well as with BA.2 Spike proteins, prepared immune libraries again, and performed phage display screening using the Omicron BA.5 RBD as a bait. We obtained three new VHH antibodies, namely Ma28D01, Ma28F04 and Ma28H11 (Tables 1 and 2).

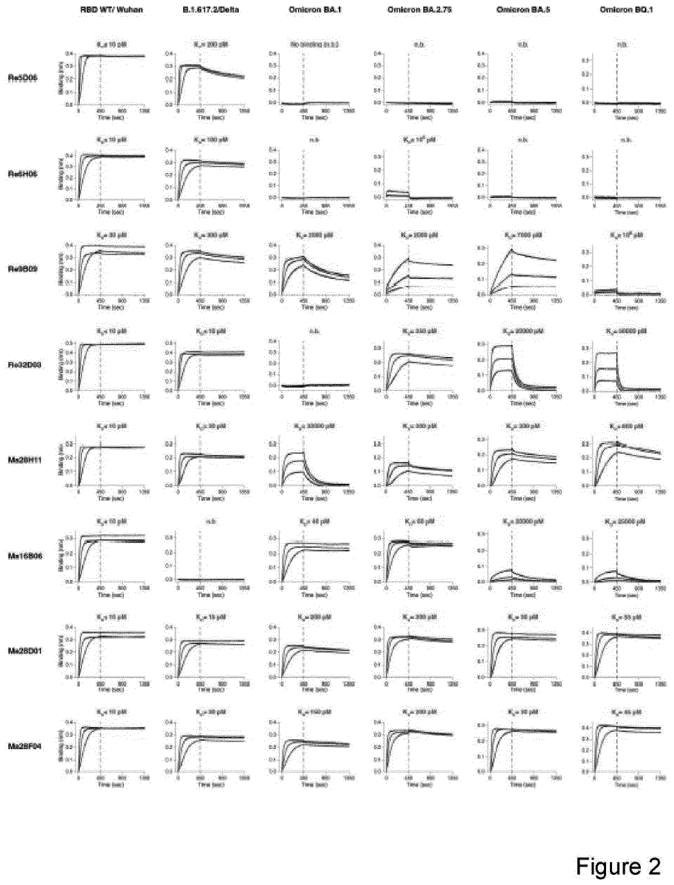

Biolayer interferometry (BLI) experiments showed that Ma28H11 had a high affinity towards Omicron BA.5 RBD with a KD of 300 pM (Table 1 and Figure 2). Ma28D01 and Ma28F04 displayed even higher binding strength (30 pM). The latter two also showed remarkable cross-reaction with RBDs of earlier VOCs, namely Wuhan/Wildtype, Delta, Omicron BA.1 as well as with Omicron BA.2.75 and BQ.1 RBDs. Ma28D01 and Ma28F04 have very similar sequences, with only one amino acid change at the CDRs (S/P at the last amino acid residue of CDR2), and two additional changes (one of them conventional E/D) at non-CDR sequences.

These new VHH antibodies were subsequently tested for Omicron BA.5 neutralization using vesicular stomatitis virus (VSV) pesudotyped virus particles. The VSV pseudotyping system employs pseudovirus particles that lack VSV envelope glycoprotein, instead incorporate protein of interest, e.g. SARS-CoV-2 Spike, at the particle surface. These particles also contain a reporter gene (e.g. green fluorescent protein, GFP) which is expressed in cells upon infection. Thus, infection and neutralization can be directly monitored using fluorescence microscopy.

Neutralization experiments revealed that Ma28D01, Ma28F04 and Ma28H11 completely neutralized pseudotyped Omicron BA.5 as low as 1 nM VHH antibody concentration (Table 1 and Figure 4). Similarly, they all cross-neutralized pseudotyped Omicron BA.2.75 as low as 1 nM concentration.

When these VHH antibodies were tested for pseudotyped Omicron BA.2 neutralization, we observed that neutralization potency of Ma28D01 and Ma28F04 were same but that of Ma28H11 was reduced, where 10 nM VHH concentration was necessary for complete neutralization.

Finally, we performed virus neutralization experiments using SARS-CoV-2 wild type virus as well as SARS-CoV-2 variants Delta and Omicron BA.1. Ma28D01, Ma28F04 and Ma28H11 all displayed cross-neutralization with wild type and Delta variant, neutralizing at 50-500 pM VHH antibody concentration (Table 1).

### VHH antibodies

The present invention relates to a VHH antibody, which is a monovalent heavy chain-only antibody comprising a CDR1 domain, a CDR2 domain and a CDR3 domain linked by framework regions including, but not being limited to, whole VHH antibodies, e.g. native VHH antibodies comprising framework regions derived from camelids, and modified VHH antibodies comprising modified framework regions, VHH antibody fragments and VHH antibody fusion proteins, e.g. a fusion protein with an immunoglobulin or non-immunoglobulin peptide or polypeptide, as long as it shows the properties according to the invention.

The present invention is also directed to a covalent or non-covalent conjugate of a VHH antibody molecule to a non-proteinaceous structure, for example, a labeling group, a capture group such a solid phase-binding group, or an effector group such as a toxin. For example, the heterologous moiety may be from a fluorescence group, biotin, an enzyme such as a peroxidase, phosphatase, or luciferase, a hapten, an affinity tag, or a nucleic acid such as an oligonucleotide.

The VHH antibody of the present invention is particularly a monoclonal VHH antibody characterized by a specific amino acid sequence. The VHH antibody may be produced in a prokaryotic host cell, a yeast cell or a mammalian cell. In certain embodiments, the VHH antibody is non-glycosylated. In certain embodiments, the VHH antibody is glycosylated, wherein a carbohydrate structure may be derived from a glycosylation site introduced into the VHH sequence and/or from a fusion partner.

A VHH antibody according to the present invention is characterized by (i) a CDR3 sequence, (ii) a combination of a CDR1 sequence, a CDR2 sequence, and a CDR3 sequence, (iii) by a complete VHH sequence, or (iv) by competition with a specific reference antibody. Specific CDR and VHH sequences are provided in the Tables, Figures and the Sequence Listing. According to the present invention, sequences related to the above sequences are encompassed. These related sequences are defined by having a minimum identity to a specifically indicated amino acid sequence, e.g., a CDR or VHH sequence. This identity is indicated over the whole length of the respective reference sequence and may be determined by using well-known algorithms such as BLAST.

In particular embodiments, a related CDR3 sequence has an identity of at least 80% or at least 90% or at least 95% to a specifically indicated CDR3 sequence, e.g., a substitution of 1, 2, or 3 amino acids.

In particular embodiments, a related combination of a CDR1 sequence, a CDR2 sequence, and a CDR3 sequence has an identity of at least 80% or at least 90% or at least 95% to a specifically indicated combination of a CDR1 sequence, a CDR2 sequence, and a CDR3 sequence, e.g., a substitution of 1, 2, 3, 4, 5 or 6 amino acids by different amino acids.

In particular embodiments, a related VHH sequence has an identity of least 70%, at least 80%, at least 90%, at least 95% or at least 99% to a VHH sequence, e.g., a substitution of 1, 2, 3, 4, 5 or up to 20 amino acids.

Further, the invention refers to a VHH antibody, which competes with a specific VHH antibody disclosed herein for the binding to the SARS-CoV-2 spike protein S1 domain. In certain embodiments, a competing VHH antibody binds the same or an overlapping epitope on the SARS-CoV-2 spike protein S1 domain, particularly the receptor-binding domain (RBD) of the SARS-CoV-2 S1 domain as a specific VHH antibody disclosed herein. For example, the invention refers to a VHH antibody, which competes either with VHH antibodies Ma28D01, Ma28F04, Ma28H11, Ma28H02, or Ma16B06.

In particular embodiments, at least one amino acid of a reference sequence, including an amino acid in a CDR1, CDR2 or CDR3 sequence and/or an amino acid in a framework region, is replaced by another amino acid, while preserving structural integrity and epitope-binding of the VHH antibody. These exchanges can be conservative (i.e., by a similar amino acid) or non-conservative.

In further particular embodiments, at least one amino acid of a reference sequence, including an amino acid in a CDR1, CDR2 or CDR3 sequence and/or an amino acid in a framework region, is replaced by a conservative amino acid substitution, i.e. a substitution of an amino acid by another amino acid with similar biochemical properties, for example a substitution of an aliphatic amino acid, e.g. Gly, Ala, Val, Leu, or Ile, for another aliphatic amino acid; a substitution of a basic amino acid, e.g. His, Lys or Arg, against another basic amino acid or against Met; a substitution of an acidic amino acid or an amide thereof, e.g., Asp, Glu, Asn or Gln, against another acidic amino acid or an amide thereof; a substitution of an aromatic amino acid, e.g., Phe, Tyr or Trp, against another aromatic amino acid.

In further particular embodiments, the VHH antibody is selected from antibody Ma28D01 comprising a VHH sequence as shown in SEQ. ID NO: 1 or a VHH antibody, which is a variant thereof. In certain embodiments, 1, 2, 3, 4, 5, 6, 7, 8, 9 or 10 amino acids of SEQ. ID NO: 1 are replaced by another amino acid. Exemplary variants comprise an amino acid sequence as shown in SEQ ID NO.30.

In further particular embodiments, the VHH antibody is selected from antibody Ma28F04 comprising a VHH sequence as shown in SEQ. ID NO: 5 or a VHH antibody, which is a variant thereof. In certain embodiments, 1, 2, 3, 4, 5, 6, 7, 8, 9 or 10 amino acids of SEQ. ID NO: 5 are replaced by another amino acid. Exemplary variants comprise an amino acid sequence as shown in SEQ ID NO.30.

In further particular embodiments, the VHH antibody is selected from antibody Ma28H11 comprising a VHH sequence as shown in SEQ. ID NO: 7 or a VHH antibody, which is a variant thereof. In certain embodiments, 1, 2, 3, 4, 5, 6, 7, 8, 9 or 10 amino acids of SEQ. ID NO: 7 are replaced by another amino acid.

In further particular embodiments, the VHH antibody is selected from antibody Ma28H02 comprising a VHH sequence as shown in SEQ. ID NO: 11 or a VHH antibody, which is a variant thereof. In certain embodiments, 1, 2, 3, 4, 5, 6, 7, 8, 9 or 10 amino acids of SEQ. ID NO: 11 are replaced by another amino acid.

In further particular embodiments, the VHH antibody is selected from antibody Ma16B06 comprising a VHH sequence as shown in SEQ. ID NO: 15 or a VHH antibody, which is a variant thereof. In certain embodiments, 1, 2, 3, 4, 5, 6, 7, 8, 9 or 10 amino acids of SEQ. ID NO: 15 are replaced by another amino acid.

Further, the present invention relates to a nucleic acid molecule, e.g., a DNA molecule, encoding a VHH as indicated above, or a vector, comprising said nucleic acid molecule as indicated above in operative linkage with an expression control sequence, particularly with a heterologous expression control sequence. Furthermore, the invention relates to a cell comprising a nucleic acid molecule or a vector as described above. Vectors for the recombinant production of VHH antibodies are well-known in the art. In certain embodiments, the vector is an extrachromosomal vector. In other embodiments, the vector is a vector for genomic integration. The cell may be a known host cell for producing antibodies or antibody fragments, e.g., a prokaryotic cell such as an E. coli or a Bacillus sp. cell, a yeast cell, particularly a *Pichia* yeast cell, an insect cell or a mammalian cell, e.g., a CHO cell, or a plant cell. In certain embodiments, the cell comprises the nucleic acid or the vector extrachromosomally. In other embodiments, the cell comprises the nucleic acid or the vector integrated into the genome, e.g., as a genomically integrated expression cassette.

Still a further aspect of the present invention is a method of recombinantly producing a VHH antibody by growing a cell as described above in a culture medium and obtaining the VHH antibody from the cell or the culture medium. Suitable culture media and culture conditions are well known in the art.

### Binding to the SARS-CoV-2 RBD

The VHH antibodies of the present invention bind to the RBD of Omicron variants of SARS-CoV-2. The inventors have identified VHH antibodies, which bind with high affinity to the Omicron BA.5 and/or BA.1 RBD as shown in Table 2, supra.

In certain embodiments, the VHH antibody of the present invention binds to the RBD of the SARS-CoV-2 Omicron BA.5 variant and to RBD of other strains and VOCs, namely Wuhan/Wildtype, Delta, Omicron BA.1 as well as Omicron BA.2.75 and BQ.1 RBDs with an affinity expressed as dissociation constant KD of about 1000 pM or less, of about 500 pM or less, of about 100 pM or less or of about 50 pM or less. The binding affinity may be determined as described herein in detail below.

### Virus neutralization

The VHH antibodies of the present invention are capable of neutralizing SARS-CoV-2 Omicron variants and subvariants thereof, e.g., the SARS-CoV-2 Omicron BA.5 and/or BA.1variant. The inventors have identified VHH antibodies, which cross-neutralize the SARS-CoV-2 Omicron BA.5 and/or BA.1 variant and other strains and variants, in particular the SARS-CoV-2 Wuhan/Wildtype, Delta, as well as Omicron BA.2.75 and BQ.1, as shown in Table 1, supra.

In certain embodiments, the VHH antibody of the present invention neutralizes SARS-CoV-2, e.g., the SARS-CoV-2 Omicron variant and subvariants thereof, in particular BA.5 as well as Omicron BA.1, BA.2.75 and BQ.1, the Wuhan/Wildtype, and the Delta variant at a concentration of about 5 nM or less, of about 1000 pM or less, of about 500 pM or less of about 170 pM or less, or of about 100 pM or less. The neutralization potency may be determined as described herein in detail below.

In particular embodiments, the VHH antibodies are capable of neutralizing a SARS-CoV2 variant comprising a spike protein RBD including at least one mutation, particularly at position G339D, S371L, S373P, S375F, K417N, N440K, G446S, S477N, T478K, E484A, Q493R, G496S, Q498R, N501Y, and/or Y505H. In even more particular embodiments, the VHH antibodies are capable of neutralizing a SARS-CoV-2 variant of concern including Omicron BA.5, BA.1, BA.2.75 and BQ.1 as well as Wuhan/Wildtype, and Delta (L452R, T478K).

In further particular embodiments, the VHH antibodies are capable of cross-neutralizing the SARS-CoV-2 Omicron BA.5 variant, and at least one of the BA.1, BA.2.75 and BQ.1 Omicron variants, the Wuhan/Wildtype, and the Delta variant. In further particular embodiments, the VHH antibodies are capable of cross-neutralizing the SARS-CoV-2 Omicron BA.5 variant, the BA.1, BA.2.75 and BQ.1 Omicron variants, the Wuhan/Wildtype, and the Delta variant. In even more particular embodiments, the VHH antibodies are capable of cross-neutralizing the SARS-CoV-2 Omicron BA.5 variant, the BA.1, BA.2.75 and BQ.1 Omicron variants, the Wuhan/Wildtype, and the Delta variant at a concentration of about 5 nM or less, of about 1000 pM or less, of about 170 pM or less, or of about 100 pM or less. The neutralization potency may be determined as described herein in detail below.

In particular embodiments, the VHH antibodies neutralize the Omicron BA.5 and/or BA1 variant of SARS-CoV-2 a concentration of about 5 nM or less, of about 1000 pM or less, of about 170 pM or less, or of about 100 pM or less, or of about 50 pM or less.

### Stability

For the intended therapeutic application, the anti-SARS-CoV-2 VHH antibodies should not only be highly potent in virus neutralization, but also, they should be developable as biological drugs. This includes that they are stable enough to survive a lengthy, large-scale production process as well as transportation and storage (ideally for years in liquid formulation) without aggregation or loss of activity.

A good predictor for stability is thermostability, which can be measured, e.g., by thermal shift assays or specifically by differential scanning fluorimetry. The present inventors have identified several thermostable or hyperthermostable VHH antibodies as shown in Table 1, supra.

In a particular embodiment, the invention relates to a VHH antibody, which is stable, particularly thermostable or hyperthermostable. Preferably, the VHH antibody has a melting point (melting temperature) of at least about 65°C, of at least about 80°C, of at least 90°C or of at least about 95°C, and/or an aggregation temperature of at least about 50°C, of at least about 60°C, of at least 70°C or of at least about 80°C when measured under non-reducing conditions. Melting and aggregation temperatures are determined as described herein.

The hyperthermostable VHH antibodies includes some extremely strong RBD-binders and very potent neutralizers, such as Ma28D01 and Ma28F04 (neutralization of the BA.1 variant at 1700 or 500 pM).

### Sets of VHH antibodies

In a further aspect, the present invention relates to a set comprising at least 2, 3, 4 or more of the above VHH antibodies. In such a set, the individual VHH antibodies are present in suitable molar ratios. Typically, the molar ratios are in the range of about 2:1 to about 1:2, particularly about 1.5:1 to about 1:1.5, even more particularly about 1:1. In certain embodiments, the VHH antibody set may comprise a single composition wherein the VHH antibodies in said set consist of a predetermined number of different species of VHH antibodies as described above. The VHH antibody set may comprise a plurality of compositions each comprising a different species of VHH antibody as described above. The set of the present invention may be free from other VHH antibodies.

Alternatively, the set of VHH antibodies may contain at least one VHH antibody described above and additionally at least one of the VHH antibodies described in PCT/EP2021/071309 and in EP21209549.1 supra.

### Heterodimeric VHH antibodies

In certain embodiments, the VHH antibody is a heterodimeric VHH antibody, particularly a covalently linked VHH heterodimer comprising a first VHH antibody and a second VHH antibody wherein the first VHH antibody and the second VHH antibody bind to different epitopes on the RBD of the SARS-CoV-2 S1 domain.

A heterodimeric VHH antibody comprises two different VHH antibodies optionally connected by linker. In particular embodiments, at least one of the VHH antibodies is a VHH antibody of the present invention as described herein. A VHH antibody of a heterodimeric VHH antibody is characterized by (i) a CDR3 sequence, (ii) a combination of a CDR1 sequence, a CDR2 sequence, and a CDR3 sequence, (iii) by a complete VHH sequence, or (iv) by competition with a specific reference antibody.

In certain embodiments, the first and the second VHH antibody do not compete with each other. Competition can be measured either as an at least 90% loss of fluorescence staining of the SARS-CoV-2 spike protein by a fluorophore-labelled candidate VHH antibody in the presence of a 100-fold molar excess of an unlabelled competitor VHH antibody, e.g., selected from Ma28D01, Ma28F04, Ma28H11, Ma28H02, or Ma16B06, or conversely as an at least 90% loss of spike protein-staining by a fluorophore-labelled VHH antibody in the presence of a 100-fold molar excess of the unlabelled candidate VHH antibody as a competitor.

Preferably, competition can be measured by label-free biolayer interferometry - performed as a cross-competition or epitope binning assay using a label-free detection system, e.g., an Octet^{®} system from Sartorius, according to the manufacturer's instructions.

The first and the second VHH antibody may be directly fused to each other or fused through a linker sequence. In particular embodiments, the first and the second VHH antibody are genetically fused by a peptidic linker sequence.

In certain embodiments, the linker is a peptidic linker, particularly having a length of 5 to 50 amino acid residues, particularly of 10 to 40 amino acid residues and more particularly of 12 to 30 amino acid residues, particularly as described in PCT/EP2021/071309 supra.

In certain embodiments, one of the first and the second VHH antibodies is a VHH antibody of the present invention as described herein and the other is a VHH antibody as described in PCT/EP2021/071309 or in EP21209549.1, for example one of the VHH antibodies described above with regard to a set of VHH antibodies.

### Homotrimeric VHH antibodies

In certain embodiments, the VHH antibody is a homotrimeric antibody.

Homotrimerization of a VHH antibody requires an appropriate polypeptide fusion partner as trimerization module. According to the present invention, a fusion partner is preferred, comprising at least one of the following features:
Preferred trimerization modules include domains derived from collagen, the most abundant class of proteins of the human body, including the trimerization (NC1) domains of collagen XV (PDB 3N3F, 54 residues) (Wirz *et al.,* 2011), collagen XVIII (PDB 3HSH; 54 residues) (Boudko *et al.,* 2009) and collagen X (PDB 1GR3; 160 amino acids) (Bogin *et al.,* 2002). Another very appropriate homotrimeric fusion partner is the lung surfactant protein D (LSFPD) that recognizes sugars on viral and bacterial pathogens (see e.g., PDB 1B08, 3IKN, 3IKQ, 3IKR). As the SARS-CoV-2 spike protein is heavily glycosylated, a VHH-LSPD fusion could confer a bimodal inhibition of spike function.

According to the present invention, it is preferred that the VHH antibody is linked to the trimerization domain via a spacer having a length of at least about 5 amino acids, particularly about 5 amino acids to about 100 amino acids, and more particularly of about 10 to about 50 amino acids, particularly as described in PCT/EP2021/071309 supra.

It should be note that different arrangements for the subunits of homotrimeric VHH antibodies are possible. As just described, the trimerization module might be located N-terminally, followed by the spacer and the VHH antibody. Alternatively, the VHH antibody might be located N-terminally, followed by spacer and trimerization module. The best arrangement will depend on the geometry of the target to be neutralized.

In particular embodiments, the present invention relates to a neutralizing homotrimeric VHH antibody, e.g., a VHH antibody which has a lowest SARS-CoV-2 neutralizing concentration of about 100 pM or less, of about 25 pM or less, of about 17 pM or less, of about 10 pM or less, of about 5 pM or less or of about 1.7 pM or less.

### Production of VHH antibodies

VHH antibodies including monomeric, heterodimeric and homomultimeric, particularly homotrimeric VHH antibodies may be produced as described in PCT/EP2021/071309 and in EP21209549.1 supra, or by other methods well known in the art.

A VHH antibody may be recombinantly produced in a suitable host cell, e.g., in a prokaryotic or eukaryotic host cell or host organism. For this purpose, a nucleic acid molecule encoding the VHH antibody is introduced into the host cell or host organism and expressed in the host cell or host organism. The nucleic acid molecule may encode a monomeric VHH antibody or a subunit of a homomultimeric VHH antibody, particularly of a homotrimeric VHH antibody.

Any therapeutic application of neutralizing anti-SARS-CoV-2 VHH antibodies requires their production at a large scale. In this aspect, production in bacteria or yeast, e.g., *Pichia pastoris, Saccharomyces cerevisiae,* or *Hansenula polymorpha,* may be preferred. Production in yeast, e.g., *Pichia pastoris, Saccharomyces cerevisiae,* or *Hansenula polymorpha,* is particularly preferred because it ensures a quantitative formation of disulfide bonds, allows for excellent yields (in the range of grams per liter culture), while the secretion into the medium allows the subsequent purification to begin with only few contaminating proteins.

In a particular embodiment, the VHH antibody is recombinantly produced in a bacterium, e.g., *E. coli or Bacillus.* For example, expression in a bacterium may involve cytoplasmic and/or periplasmic expression and purification of the VHH antibody from the host cell, or secretory expression and purification of the VHH antibody from the culture medium. In certain embodiments, the nucleic acid sequence encoding the VHH antibody is fused to at least one sequence directing the expression to the periplasm and/or into the culture medium.

In a further particular embodiment, the VHH antibody is recombinantly produced in a eukaryotic host cell or host organism, preferably in yeast, e.g., in *Pichia pastoris, Saccharomyces cerevisiae,* or *Hansenula polymorpha.* For example, expression in a eukaryotic host cell or host organism, e.g., yeast may involve cytoplasmic and/or periplasmic expression and purification of the VHH antibody from the host cell, or preferably secretion from the host cell and purification of the VHH antibody from the culture medium. In certain embodiments, the nucleic acid sequence encoding the VHH antibody is fused to at least one sequence directing the expression into the culture medium.

The VHH antibody encoding nucleic acid molecule may comprise a sequence encoding a monomeric VHH antibody, a sequence encoding a heterodimeric VHH antibody or a sequence encoding a subunit of a multimeric VHH antibody, particularly of a trimeric VHH antibody. The sequence encoding a heterodimeric VHH antibody may comprise two sequences each encoding a monomeric VHH antibody arranged in tandem fused to each other optionally through a linker. The subunit sequence may comprise (from N-terminus to C-terminus) the VHH antibody sequence, optionally a spacer, and a multimerization domain, particularly a trimerization domain, e.g., the Collagen XVIII NC1 domain. Alternatively, the subunit sequence may comprise (from N-terminus to C-terminus) a multimerization domain, particularly a trimerization domain, optionally a spacer and the VHH antibody sequence.

### Therapeutic applications

Still a further aspect of the present invention is the use of an active agent as described above selected from a monomeric VHH antibody, a heterodimeric VHH antibody, a set of at least 2 different VHH antibodies, or a multimeric, particularly a trimeric VHH antibody in medicine, particularly for therapeutic and/or in vitro or vivo diagnostic use. In certain embodiments, the monomeric VHH antibody, the heterodimeric VHH antibody, the set of VHH antibodies or the multimeric, particularly trimeric VHH antibody is used in human medicine.

In particular embodiments, a therapeutic VHH antibody is selected from (i) a VHH antibody comprising the CDR3 sequence of the thermostable VHH antibody Ma28D01 or a related sequence, (ii) a VHH antibody comprising the CDR1, CDR2 and CDR3 sequences of the VHH antibody Ma28D01 or related sequences, or (iii) a VHH antibody comprising the VHH sequence of the VHH antibody Ma28D01 or a related sequence. In certain embodiments, 1, 2, 3, 4, 5, 6, 7, 8, 9 or 10 amino acids of the VHH sequence of the reference antibody are replaced by another amino acid.

In particular embodiments, a therapeutic VHH antibody is selected from (i) a VHH antibody comprising the CDR3 sequence of the thermostable VHH antibody Ma28F04 or a related sequence, (ii) a VHH antibody comprising the CDR1, CDR2 and CDR3 sequences of the VHH antibody Ma28F04or related sequences, or (iii) a VHH antibody comprising the VHH sequence of the VHH antibody Ma28F04or a related sequence. In certain embodiments, 1, 2, 3, 4, 5, 6, 7, 8, 9 or 10 amino acids of the VHH sequence of the reference antibody are replaced by another amino acid.

In particular embodiments, a therapeutic VHH antibody is selected from (i) a VHH antibody comprising the CDR3 sequence of the thermostable VHH antibody Ma28H11 or a related sequence, (ii) a VHH antibody comprising the CDR1, CDR2 and CDR3 sequences of the VHH antibody Ma28H11 or related sequences, or (iii) a VHH antibody comprising the VHH sequence of the VHH antibody Ma28H11or a related sequence. In certain embodiments, 1, 2, 3, 4, 5, 6, 7, 8, 9 or 10 amino acids of the VHH sequence of the reference antibody are replaced by another amino acid.

In particular embodiments, a therapeutic VHH antibody is selected from (i) a VHH antibody comprising the CDR3 sequence of the thermostable VHH antibody Ma28H02 or a related sequence, (ii) a VHH antibody comprising the CDR1, CDR2 and CDR3 sequences of the VHH antibody Ma28H02 or related sequences, or (iii) a VHH antibody comprising the VHH sequence of the VHH antibody Ma28H02 or a related sequence. In certain embodiments, 1, 2, 3, 4, 5, 6, 7, 8, 9 or 10 amino acids of the VHH sequence of the reference antibody are replaced by another amino acid.

In particular embodiments, a therapeutic VHH antibody is selected from (i) a VHH antibody comprising the CDR3 sequence of the thermostable VHH antibody Ma16B06 or a related sequence, (ii) a VHH antibody comprising the CDR1, CDR2 and CDR3 sequences of the VHH antibody Ma16B06 or related sequences, or (iii) a VHH antibody comprising the VHH sequence of the VHH antibody Ma16B06 or a related sequence. In certain embodiments, 1, 2, 3, 4, 5, 6, 7, 8, 9 or 10 amino acids of the VHH sequence of the reference antibody are replaced by another amino acid.

Therapeutic applications of an active agent as described above, e.g., a monomeric VHH antibody, a heterodimeric VHH antibody, a set of at least 2 different VHH antibodies, or a multimeric, particularly trimeric VHH antibody include methods for preventing or treating a disorder caused by and/or associated with a Coronavirus infection, particularly a disorder caused by and/or associated with a SARS-CoV-2 infection and more particularly of preventing or treating COVID-19. The therapeutic applications also include methods for preventing or treating a disorder caused by and/or associated with an infection by a SARS-CoV-2 Omicron variant including the BA.5, BA.1, BA.2, BA.2.75, BA.4, and BQ.1. variant as well as variants comprising at least one of the amino acid substitutions in the RBD domain in any one of the above variants.

In therapeutic applications, the active agent is administered in an effective amount to a subject in need thereof, particularly to a human subject. The dose will depend on the specific type of agent, e.g. monovalent VHH antibody or multimeric VHH antibody, and the type of disease.

In the prevention or treatment of COVID-19, a daily dose of a monomeric VHH antibody for a human subject is any suitable dose based on the severity of the disease, the specific type of agent, and the mode of administration, e.g., locally, or systemically, particularly from about 5 µg to about 5000 mg per day.

In the prevention or treatment of COVID-19, a daily dose of a heterodimeric VHH antibody for a human subject is any suitable dose based on the severity of the disease, the specific type of agent, and the mode of administration, e.g., locally, or systemically, particularly from about 5 µg to about 5000 mg per day.

In the prevention or the treatment of COVID-19, a daily dose of a trimeric VHH antibody for a human subject is any suitable dose based on the severity of the disease, the specific type of agent, and the mode of administration, e.g., locally or systemically, particularly from about 1 µg to about 2500 mg per day.

In the prevention or treatment of COVID-19, a unit dose of a monomeric VHH antibody for a human subject is any suitable dose based on the severity of the disease, the specific type of agent, and the mode of administration, e.g., locally, or systemically, particularly from about 5 µg to about 5000 mg per unit.

In the prevention or treatment of COVID-19, a unit dose of a heterodimeric VHH antibody for a human subject is any suitable dose based on the severity of the disease, the specific type of agent, and the mode of administration, e.g., locally, or systemically, particularly from about 5 µg to about 5000 mg per unit.

In the prevention or the treatment of COVID-19, a unit dose of a trimeric VHH antibody for a human subject is any suitable dose based on the severity of the disease, the specific type of agent, and the mode of administration, e.g., locally, or systemically, particularly from about 1 µg to about 2500 mg per unit.

Typically, the VHH antibody is administered as a pharmaceutical composition comprising the active agent and a pharmaceutically acceptable carrier or excipient. Examples of suitable carriers and excipients for formulating antibodies or antibody fragments are well-known in the art.

Depending on the stage and the severity of the disorder, the pharmaceutical composition may be administered once or several times during the course of the disorder. For example, it may be administered once or several times daily, each second day, two times weekly or weekly for a suitable period of time.

In certain embodiments, the pharmaceutical composition is administered parenterally, e.g., by subcutaneous, intramuscular or intravenous injection or by infusion. In further embodiments, the pharmaceutical composition may be administered locally, e.g., orally, nasally or intrapulmonary, for example by inhalation as an aerosol.

In certain embodiments, the pharmaceutical composition is administered in an early infection stage, e.g., in an infection stage where the upper airways such as the oral cavity, the nasal cavity, the paranasal sinus, the pharynx and/or the throat are infected, but the lower airways such as the bronchi and/or the lung are not infected. In further embodiments, the pharmaceutical composition is administered in a late infection stage wherein the lower airways such as the bronchi and/or the lung are infected. Further, the pharmaceutical composition may be administered in an infection stage where the subject suffers from a respiratory dysfunction and optionally is ventilated. In still further embodiments, the pharmaceutical composition may be administered prophylactically to a subject being at risk of a Coronavirus infection, e.g., a subject, which has previously, for example, within 1, 2, 3, 4, or 5 days or even longer, been in close contact with an infected subject.

The VHH antibody may be administered alone or together with a further active agent, which may be selected from anti-viral agents such as remdesivir, methotrexate, Molnupiravir, or Paxlovid (Wang *et al.,* 2020b; Malone and Campbell, 2021; Stegmann *et al.*, 2021).

A further aspect that needs to be considered is the plasma half-life of a VHH antibody. Proteins smaller than 40 kDa are subject to considerable renal clearance, and an isolated VHH antibody (-15 kDa) shows, therefore, a plasma half-life of only 4 hours (Hoefman *et al.,* 2015). Thus, the administration might comprise repeated daily injections or continuous perfusion during anti-viral therapy that might last for days to weeks. One solution to this problem is to increase the size of the protein. Fusion to spacer and trimerization module increases the size to ≥70 kDa, and such fusion should then have a far longer plasma half-life that is no longer limited by renal clearance.

Further, fusion to Fc fragments resulting in a VHH-Fc fusion protein significantly increases the protective activity in vivo and plasma half-life. An alternative strategy for half-life extension is to exploit the long circulating half-life of serum albumin by fusing the VHH antibodies of the invention to serum albumin or to a binding moiety that recognizes albumin.

One can, however, also imagine alternative application routes. In fact, inhalation of an anti-SARS-CoV-2 VHH antibody formulation would bring the therapeutic agent directly to the site of the highest and most problematic virus load, namely the airway epithelia.

The here outlined antiviral clinical strategy will become more robust if not just one but an entire set of several VHH antibodies is deployed. First, one should expect synergistic effects for neutralizing antibodies that bind to non-overlapping sites. Second, patients might develop anti-idiotypic antibodies against a given therapeutic VHH antibody and render it ineffective in treatment. A switch to another VHH would solve the problem. Third, and perhaps most importantly there is the clear risk that SARS-CoV2 evolves further, that escape mutations in its RBD might destroy the epitope of a given VHH antibody and render such VHH inefficient in therapy. This risk can be minimized by utilizing a panel of neutralizing antibodies that recognize non-identical and possibly even non-overlapping epitopes, or by at least switching to another VHH that still neutralizes a given escape mutant.

### Diagnostic applications

Diagnostic applications include in vitro methods wherein a VHH antibody is used for detecting Coronavirus spike protein in a sample, e.g., in a body fluid such as saliva, blood, serum or plasma, stool samples or in a tissue or biopsy sample. Diagnostic applications further include in vivo methods wherein a VHH antibody is used for detecting Coronavirus spike protein in a subject, particularly in a human patient. For diagnostic applications the VHH antibody carry a label for direct detection or used in combination with secondary detection reagents, e.g., antibodies, including conventional antibodies or VHH antibodies, for indirect detection according to established techniques in the art.

Further, the present invention is explained in more detail by the following Figures and Examples.

### Figure legends

- **Figure 1:**: Binding of VHH antibodies to the RBD of the SARS-CoV-2 Omicron BA.1 variant
- **Figure 2:**: Affinity measurements of VHH antibodies against SARS-CoV-2 wild type and variants RBDs Fitted curves and calculated dissociation constants (KDs) are shown in red. Where curve fitting failed due to low or no binding signal, sensograms are denoted as "no binding" (n.b.) in red.
- **Figure 3:**: Neutralization of the SARS-CoV-2 Omicron BA.1 variant by Ma16B06
- **Figure 4:**: Neutralizing activity of VHH antibodies against pseudotyped SARS-CoV-2 Omicron variants.
- **Figure 5:**: Thermostability of anti-Omicron VHH antibodies

### VHH antibody sequences (comprising the CDRs shown in Table 1)

>Re9H03 (SEQ ID NO. 19) 
QVQLVESGGGLVQPGGSLRLSCAASGFTLDYYAIGWFRQAPGKEREGVSRISSSD
GSTDYADSVKGRFTISRDNAKNTVYLQMNSLKPEDTAVYYCATVPGTYYSGNWYY
TWHPKAVDYWGKGTLVTVSS
>Ma16B06 (SEQ ID NO. 15) 
QVQLVESGGGLVRTGGSLRLSCAASGSILQIWAMKWYRQAPGLQREWIATIPNSG
EPFYASSVEGRFTGSRENEETVYLYLNNLEPEDTAVYYCEVNEGVPVREYWGQGT
QVTVSS
>Ma28H02 (SEQ ID NO. 11) 
QVQLVESGGGLVQPGGSLRLSCATSEIGLSYYGVGWFLQAPGKDREGVACMHNT
DGNPVYAPSVKGRFTISRDDAKKMVYLEMNNLKPEDTGVYYCAVGPLSPGITCRIP
TTLGYDDWGQGTQVTVSS
>Ma28H11 (SEQ ID NO. 7) 
QVQLVESGGGLVQPGGSLRLSCATSGLTLDHYAIGWFLQAPGKEREGVACVSDDE
RSLYAPSVMGRFSISRDNAKKTFYLEMNSLKPEDTGVYYCASGPVVPGTLCRIPTV
LGYDDWGQGTQVTVSS
>Ma28D01 (SEQ ID NO. 1) 
QVQLVESGGGLVQPGGSLRLSCVVSELGLDYYQIGWFRQAPGKEREGVSCFRSS
DEIRYYADSVKGRFTTFRDNAGTTVYLQMNSLKPEDTGVYYCAVAPQTYYHGTWY
GECAAAGMDSWGKGTQVTVSS
>Ma28F04 (SEQ ID NO. 5)

QVQLVESGGGLVQPGGSLRLSCVVSDLGLDYYQIGWFRQAPGKEREGVSCFRSS
DEIRYYADPVKGRFTTFRDNAGTTVYLQMNSLKPEDTGVYYCAVAPQTYYHGTWY
GECAAAGMDSWGKGTLVTVSS

### RBD Sequences

>wt_RBD-AviHis (SEQ ID NO. 23) 
ETNLCPFGEVFNATRFASVYAWNRKRISNCVADYSVLYNSASFSTFKCYGVSPTKL
NDLCFTNVYADSFVIRGDEVRQIAPGQTGKIADYNYKLPDDFTGCVIAWNSNNLDS
KVGGNYNYLYRLFRKSNLKPFERDISTEIYQAGSTPCNGVEGFNCYFPLQSYGFQP
TNGVGYQPYRVVVLSFELLHAPATVCGPKTGTGGSGGSGLNDIFEAQKIEWHEGR
TKHHHHHH
>Delta_RBD-Avi-His (SEQ ID NO. 24) 
ETNLCPFGEVFNATRFASVYAWNRKRISNCVADYSVLYNSASFSTFKCYGVSPTKL
NDLCFTNVYADSFVIRGDEVRQIAPGQTGKIADYNYKLPDDFTGCVIAWNSNNLDS
KVGGNYNYRYRLFRKSNLKPFERDISTEIYQAGSKPCNGVEGFNCYFPLQSYGFQP
TNGVGYQPYRVVVLSFELLHAPATVCGPKTGTGGSGGSGLNDIFEAQKIEWHEGR
TKHHHHHH
>Omicron_BA.1_RBD-AviHis (SEQ ID NO. 25) 
ETGNLCPFDEVFNATRFASVYAWNRKRISNCVADYSVLYNLAPFFTFKCYGVSPTK
LNDLCFTNVYADSFVIRGDEVRQIAPGQTGNIADYNYKLPDDFTGCVIAWNSNKLDS
KVSGNYNYLYRLFRKSNLKPFERDISTEIYQAGNKPCNGVAGFNCYFPLRSYSFRP
TYGVGHQPYRVVVLSFELLHAPATVCGPKGTGGSGGSGLNDIFEAQKIEWHEGGS
GRSHHHHSGHHHTGHHHHSGSHHH
>Omicron_BA.5_RBD-AviHis (SEQ ID NO. 26) 
ETGNLCPFDEVFNATRFASVYAWNRKRISNCVADYSVLYNFAPFFAFKCYGVSPTK
LNDLCFTNVYADSFVIRGNEVSQIAPGQTGNIADYNYKLPDDFTGCVIAWNSNKLDS
 
KVGGNYNYRYRLFRKSNLKPFERDISTEIYQAGNKPCNGVAGVNCYFPLQSYGFR
PTYGVGHQPYRVVVLSFELLHAPATVCGPKGTGGSGGSGLNDIFEAQKIEWHEGG
SGRSHHHHSGHHHTGHHHHSGSHHH
>Omicron_BA.2.75_RBD-AviHis (SEQ ID NO. 27)

ETGNLCPFHEVFNATRFASVYAWNRKRISNCVADYSVLYNFAPFFAFKCYGVSPTK
LNDLCFTNVYADSFVIRGNEVSQIAPGQTGNIADYNYKLPDDFTGCVIAWNSNKLDS
KVSGNYNYLYRLFRKSKLKPFERDISTEIYQAGNKPCNGVAGFNCYFPLQSYGFRP
TYGVGHQPYRVVVLSFELLHAPATVCGPKGTGGSGGSGLNDIFEAQKIEWHEGGS
GRSHHHHSGHHHTGHHHHSGSHHH

### Examples

### Example 1 Binding of VHH antibodies to the RBD of the SARS-CoV-2 Omicron BA.1 variant

BLI experiments were performed for the indicated VHH antibodies with biotinylated Omicron BA.1 RBD (40592-V49H7-B, Sino Biological) or Delta RBD. VHH antibodies failed binding to the BA.1 RBD at the tested concentration. In contrast, they bound the Delta RBD rapidly and with a non-detectable dissociation rate. Results are shown in Fig. 1.

### Example 2 Affinity measurements of VHH antibodies against SARS-CoV-2 wild type and variants RBDs

BLI experiments were performed for the indicated VHH antibodies with biotinylated wild type RBD, Delta RBD, Omicron BA.1 RBD (40592-V49H7-B, Sino Biological), Omicron BA.2.75 RBD, Omicron BA.5 RBD or Omicron BQ.1 RBD. Curves (black) were fitted with a mass transport model. The results are shown in Fig. 2.

### Example 3 Neutralization of the SARS-CoV-2 Omicron BA.1 variant by Ma16B06

Vero E6 cells were grown in 96 well plates and infected with the SARS-CoV-2 Omicron BA.1 variant that was pre-incubated with indicated concentrations ov VHH Re32D03 or Ma16B06. Cells were fixed 48 h later, permeabilized and stained with DAPI (to locate cell nuclei) and a cocktail of fluorescently labelled VHH antibodies that cross-react with the SARS-CoV-2 Spike of a broad range of variants (Re9H03, Re7E02, Re9C07, Re9G12, Re22D04, Re6D06, Re8H11, Ma3F05, and Ma12A03 as disclosed in co-owned application PCT/EP2021/071309 and in EP21209549.1 supra). Analysis was by automated spinning disc fluorescence microscopy.

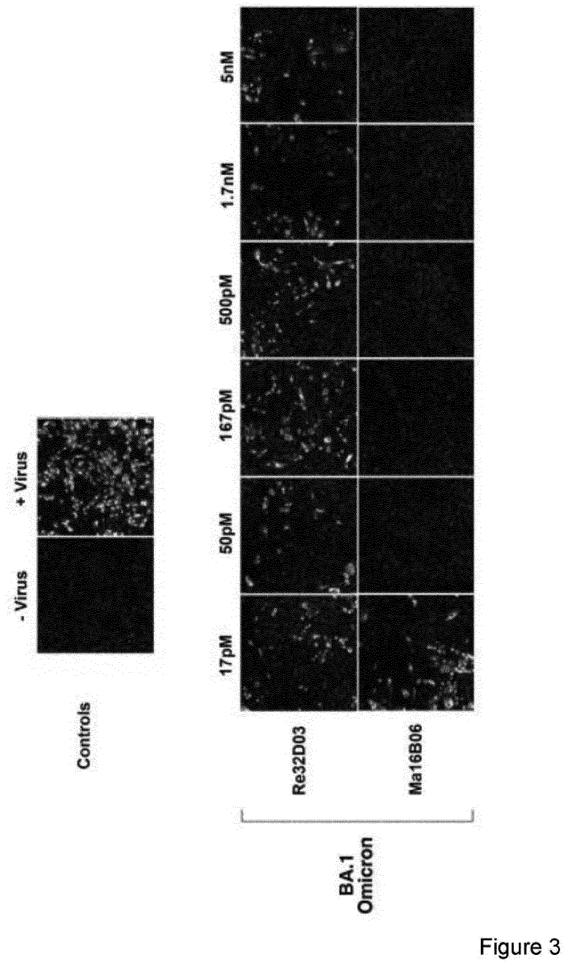

With a lowest neutralizing concentration of 50 pM, Ma16B06 showed great neutralization potency. Results are shown in Fig. 3.

### Example 4 Neutralizing activity of VHH antibodies against pseudotyped SARS-CoV-2 Omicron variants.

SARS-CoV-2 pseudotype particles indicated in Fig. 4 were pre-incubated with VHH antibodies and later used to infect Vero E6 cells that were grown in 96 well plates. After 24 h, cells were imaged on a Celigo whole-well image cytometer in green channel. Pseudotype particles encode a GFP reporter gene that is expressed in infected cells. Neutralization potency was calculated by comparing the number of infected (green fluorescent) cells in VHH antibody pre-incubated wells with that of a control (no VHH antibody pre-incubation). Results are shown in Fig. 4.

### Example 5 Thermostability of anti-Omicron VHH antibodies

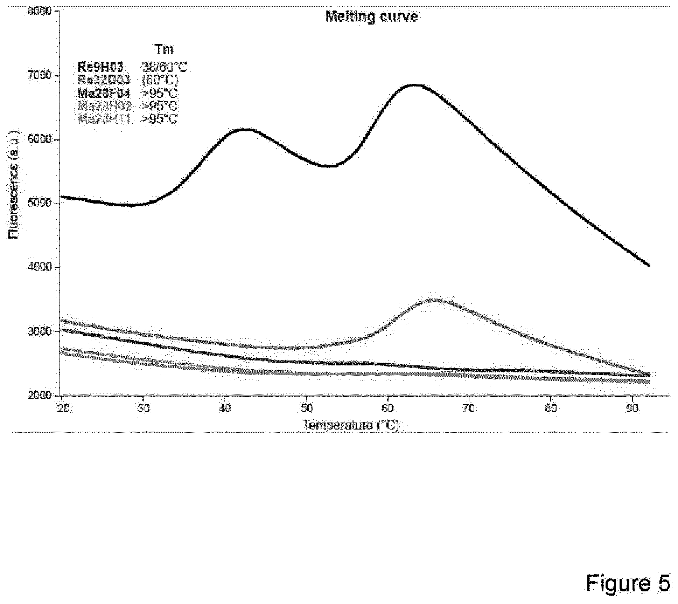

VHH antibodies indicated in Fig. 5 were subjected to Differential scanning fluorimetry (DSF), which exploits that thermal unfolding exposes aromatic/hydrophobic residues, which then bind and enhance fluorescence of the added SYPRO orange dye. Two replicates of each sample were placed onto a Thermal Cycler. The samples were incubated for 5 min at 25 °C and then the temperature was increased in 1 °C increments of 45 seconds to 95 °C. Fluorescence was measured at the end of each step.

Results are shown in Fig. 5.

The flat fluorescence curves of Ma28F04, Ma28H02, and M28H11 indicate that no melting occurred when heated to 95°C. These VHH antibodies also resisted aggregation.

### References

Arbabi Ghahroudi M, Desmyter A, Wyns L, Hamers R, Muyldermans S (1997) Selection and identification of single domain antibody fragments from camel heavy-chain antibodies. FEBS Lett, 414: 521-526
Bogin O, Kvansakul M, Rom E, Singer J, Yayon A, Hohenester E (2002) Insight into Schmid metaphyseal chondrodysplasia from the crystal structure of the collagen X NC1 domain trimer. Structure, 10: 165-173
Boudko SP, Sasaki T, Engel J, Lerch TF, Nix J, Chapman MS, Bächinger HP (2009) Crystal structure of human collagen XVIII trimerization domain: A novel collagen trimerization Fold. J Mol Biol, 392: 787-802
Chen X, Zhao B, Qu Y, Chen Y, Xiong J, Feng Y, Men D, Huang Q, Liu Y, Yang B, Ding J, Li F (2020) Detectable serum SARS-CoV-2 viral load (RNAaemia) is closely correlated with drastically elevated interleukin 6 (IL-6) level in critically ill COVID-19 patients. Clin Infect Dis*,*
Chug H, Trakhanov S, Hülsmann BB, Pleiner T, Görlich D (2015) Crystal structure of the metazoan Nup62•Nup58•Nup54 nucleoporin complex. Science, 350: 106-110
Clark SA, Clark LE, Pan J, Coscia A, McKay LGA, Shankar S, Johnson RI, Brusic V, Choudhary MC, Regan J, Li JZ, Griffiths A, Abraham J (2021) SARS-CoV-2 evolution in an immunocompromised host reveals shared neutralization escape mechanisms. Cell, S0092-8674(21)00355
Corman VM, Landt O, Kaiser M, Molenkamp R, Meijer A, Chu DK, Bleicker T, Brünink S, Schneider J, Schmidt ML, Mulders DG, Haagmans BL, van der Veer B, van den Brink S, Wijsman L, Goderski G, Romette JL, Ellis J, Zambon M, Peiris M, Goossens H, Reusken C, Koopmans MP, Drosten C (2020) Detection of 2019 novel coronavirus (2019-nCoV) by real-time RT-PCR. Euro Surveill, 25**:**
Duan K, Liu B, Li C, Zhang H, Yu T, Qu J, Zhou M, Chen L, Meng S, Hu Y, Peng C, Yuan M, Huang J, Wang Z, Yu J, Gao X, Wang D, Yu X, Li L, Zhang J, Wu X, Li B, Xu Y, Chen W, Peng Y, Hu Y, Lin L, Liu X, Huang S, Zhou Z, Zhang L, Wang Y, Zhang Z, Deng K, Xia Z, Gong Q, Zhang W, Zheng X, Liu Y, Yang H, Zhou D, Yu D, Hou J, Shi Z, Chen S, Chen Z, Zhang X, Yang X (2020) Effectiveness of convalescent plasma therapy in severe COVID-19 patients. Proceedings of the National Academy of Sciences, 117: 9490-9496
Garcia-Beltran WF, Lam EC, St Denis K, Nitido AD, Garcia ZH, Hauser BM, Feldman J, Pavlovic MN, Gregory DJ, Poznansky MC, Sigal A, Schmidt AG, Iafrate AJ, Naranbhai V, Balazs AB (2021) Multiple SARS-CoV-2 variants escape neutralization by vaccine-induced humoral immunity. Cell, 184: 2372-2383.e9
Hoefman S, Ottevaere I, Baumeister J, Sargentini-Maier M (2015) Pre-Clinical Intravenous Serum Pharmacokinetics of Albumin Binding and Non-Half-Life Extended Nanobodies®. Antibodies, 4: 141-156
Hoffmann M, Arora P, Groβ R, Seidel A, Hörnich BF, Hahn AS, Krüger N, Graichen L, Hofmann-Winkler H, Kempf A, Winkler MS, Schulz S, Jäck HM, Jahrsdörfer B, Schrezenmeier H, Müller M, Kleger A, Münch J, Pöhlmann S (2021) SARS-CoV-2 variants B.1.351 and P.1 escape from neutralizing antibodies. Cell, 184: 2384-2393.e12
Kliks SC, Nisalak A, Brandt WE, Wahl L, Burke DS (1989) Antibody-dependent enhancement of dengue virus growth in human monocytes as a risk factor for dengue hemorrhagic fever. Am J Trop Med Hyg, 40: 444-451
Li Q, Nie J, Wu J, Zhang L, Ding R, Wang H, Zhang Y, Li T, Liu S, Zhang M, Zhao C, Liu H, Nie L, Qin H, Wang M, Lu Q, Li X, Liu J, Liang H, Shi Y, Shen Y, Xie L, Zhang L, Qu X, Xu W, Huang W, Wang Y (2021) SARS-CoV-2 501Y.V2 variants lack higher infectivity but do have immune escape. Cell, 184: 2362-2371.e9
Littaua R, Kurane I, Ennis FA (1990) Human IgG Fc receptor II mediates antibody-dependent enhancement of dengue virus infection. J Immunol, 144: 3183-3186
Magro C, Mulvey JJ, Berlin D, Nuovo G, Salvatore S, Harp J, Baxter-Stoltzfus A, Laurence J (2020) Complement associated microvascular injury and thrombosis in the pathogenesis of severe COVID-19 infection: A report of five cases. Transl Res, 220: 1-13
Malone B, Campbell EA (2021) Molnupiravir: coding for catastrophe. Nat Struct Mol Biol, 28: 706-708
Mehta P, McAuley DF, Brown M, Sanchez E, Tattersall RS, Manson JJ, HLH Across Speciality Collaboration UK (2020) COVID-19: consider cytokine storm syndromes and immunosuppression. Lancet, 395: 1033-1034
Murphy K, Weaver C. (2016) Janeway's Immunobiology. Garland Science,
Pleiner T, Bates M, Görlich D (2018) A toolbox of anti-mouse and anti-rabbit IgG secondary nanobodies. J Cell Biol, 217: 1143-1154
Pleiner T, Bates M, Trakhanov S, Lee CT, Schliep JE, Chug H, Böhning M, Stark H, Urlaub H, Görlich D (2015) Nanobodies: site-specific labeling for super-resolution imaging, rapid epitope-mapping and native protein complex isolation. Elife, 4: e11349
Rasmussen SG, Choi HJ, Fung JJ, Pardon E, Casarosa P, Chae PS, Devree BT, Rosenbaum DM, Thian FS, Kobilka TS, Schnapp A, Konetzki I, Sunahara RK, Gellman SH, Pautsch A, Steyaert J, Weis WI, Kobilka BK (2011) Structure of a nanobody-stabilized active state of the β(2) adrenoceptor. Nature, 469: 175-180
Starr TN, Greaney AJ, Addetia A, Hannon WW, Choudhary MC, Dingens AS, Li JZ, Bloom JD (2021) Prospective mapping of viral mutations that escape antibodies used to treat COVID-19. Science, 371: 850-854
Stegmann KM, Dickmanns A, Gerber S, Nikolova V, Klemke L, Manzini V, Schlösser D, Bierwirth C, Freund J, Sitte M, Lugert R, Salinas G, Meister TL, Pfaender S, Görlich D, Wollnik B, Groβ U, Dobbelstein M (2021) The folate antagonist methotrexate diminishes replication of the coronavirus SARS-CoV-2 and enhances the antiviral efficacy of remdesivir in cell culture models. Virus Res, 302: 198469
Tada T, Dcosta BM, Zhou H, Vaill A, Kazmierski W, Landau NR (2021) Decreased neutralization of SARS-CoV-2 global variants by therapeutic anti-spike protein monoclonal antibodies. bioRxiv, 2021.02.18.431897
Wang C, Li W, Drabek D, Okba NMA, van Haperen R, Osterhaus ADME, van Kuppeveld FJM, Haagmans BL, Grosveld F, Bosch BJ (2020a) A human monoclonal antibody blocking SARS-CoV-2 infection. Nat Commun, 11: 2251
Wang Y, Zhang D, Du G, Du R, Zhao J, Jin Y, Fu S, Gao L, Cheng Z, Lu Q, Hu Y, Luo G, Wang K, Lu Y, Li H, Wang S, Ruan S, Yang C, Mei C, Wang Y, Ding D, Wu F, Tang X, Ye X, Ye Y, Liu B, Yang J, Yin W, Wang A, Fan G, Zhou F, Liu Z, Gu X, Xu J, Shang L, Zhang Y, Cao L, Guo T, Wan Y, Qin H, Jiang Y, Jaki T, Hayden FG, Horby PW, Cao B, Wang C (2020b) Remdesivir in adults with severe COVID-19: a randomised, double-blind, placebo-controlled, multicentre trial. Lancet, 395: 1569-1578
Wibmer CK, Ayres F, Hermanus T, Madzivhandila M, Kgagudi P, Oosthuysen B, Lambson BE, De Oliveira T, Vermeulen M, Van der Berg K (2021) SARS-CoV-2 501Y. V2 escapes neutralization by South African COVID-19 donor plasma. Nature medicine, 27: 622-625
Wirz JA, Boudko SP, Lerch TF, Chapman MS, Bächinger HP (2011) Crystal structure of the human collagen XV trimerization domain: A potent trimerizing unit common to multiplexin collagens. Matrix Biology, 30: 9-15
Zhou D, Dejnirattisai W, Supasa P, Liu C, Mentzer AJ, Ginn HM, Zhao Y, Duyvesteyn HME, Tuekprakhon A, Nutalai R, Wang B, Paesen GC, Lopez-Camacho C, Slon-Campos J, Hallis B, Coombes N, Bewley K, Charlton S, Walter TS, Skelly D, Lumley SF, Dold C, Levin R, Dong T, Pollard AJ, Knight JC, Crook D, Lambe T, Clutterbuck E, Bibi S, Flaxman A, Bittaye M, Belij-Rammerstorfer S, Gilbert S, James W, Carroll MW, Klenerman P, Barnes E, Dunachie SJ, Fry EE, Mongkolsapaya J, Ren J, Stuart DI, Screaton GR (2021) Evidence of escape of SARS-CoV-2 variant B.1.351 from natural and vaccine-induced sera. Cell, 184: 2348-2361.e6

## Claims

1. A VHH antibody recognizing the SARS-CoV-2 spike protein S1 domain, particularly the receptor-binding domain (RBD) of the SARS-CoV-2 S1 domain comprising
(a) a CDR3 sequence as shown in SEQ. ID NO: 4, 10, 14, or 18, or
(b) a CDR3 sequence, which has an identity of at least 80%, at least 90% or at least 95% to a CDR3 sequence of (a).

2. The VHH antibody according to claim 1 comprising
(a) a combination of CDR1, CDR2 and CDR3 sequences as shown in SEQ. ID NO: 2-4 or 2, 6 and 4; 8-10; 12-14; or 16-18, or
(b) a combination of CDR1, CDR2 and CDR3 sequences which has an identity of at least 80%, at least 90% or at least 95% to a combination of CDR1, CDR2 and CDR3 sequences of (a).

3. The VHH antibody according to claim 1 or 2 comprising
(a) a VH sequence as shown in SEQ. ID NO: 1, 5, 7, 11, or 15, or
(b) a sequence which has an identity of at least 70%, at least 80%, at least 90%, at least 95% or at least 99% to a VHH sequence of (a).

4. The VHH antibody of any one of claims 1-3, which binds to the RBD of the SARS-CoV-2 Omicron Variant BA.5 (comprised in SEQ ID NO: 26) and/or BA.1 (comprised in SEQ ID NO; 125) with an affinity expressed as dissociation constant KD of about 1000 pM or less, of about 500 pM or less, of about 100 pM or less or of about 50 pM or less.

5. The VHH antibody of any one of claims 1-4, which neutralizes SARS-CoV-2, e.g., the SARS-CoV-2 Omicron Variant or a subvariant thereof, e.g. BA.5, BA.1, BA.2.75, BQ.1 and the Wuhan strain, and/or a SARS-CoV-2 variant, particularly a variant of concern including Alpha (N501Y), Beta (N501Y, E484K, K417N), Gamma (N501Y, E484K, K417T), Delta (L452R, T478K), Epsilon (L452R) and/or Mu (E484K, N501Y) at a concentration of about 5 nM or less, of about 1000 pM or less, of about 500 pM or less, of about 170 pM or less, or of about 100 pM or less.

6. The VHH antibody of claim 5, which cross-neutralizes the SARS-CoV-2 Omicron Variants BA.5, BA.1, BA.2.75 and BQ.1, the Wuhan strain, and the Delta variant, particularly at a concentration of about 5 nM or less, of about 1000 pM or less, of about 500 pM or less, of about 170 pM or less, or of about 100 pM or less.

7. The VHH antibody of any one of claims 1-6, which has a melting temperature of at least about 65°C, of at least about 80°C, of at least 90°C or of at least about 95°C when measured under non-reducing conditions, and/or which has an aggregation temperature of at least about 50°C, of at least about 60°C, of at least 70°C or of at least about 80°C when measured under non-reducing conditions.

8. The VHH antibody of any one of claims 1-7, which is selected from:
(i) VHH antibody Ma28D01 comprising a VHH sequence as shown in SEQ. ID NO: 1 or a VHH antibody, which is a variant thereof,
(ii) VHH antibody Ma28F04 comprising a VHH sequence as shown in SEQ. ID NO: 5 or a VHH antibody, which is a variant thereof,
(iii) VHH antibody Ma28H11 comprising a VHH sequence as shown in SEQ. ID NO: 7 or a VHH antibody, which is a variant thereof,
(iv) VHH antibody Ma28H02 comprising a VHH sequence as shown in SEQ. ID NO: 11 or a VHH antibody, which is a variant thereof, and
(v) VHH antibody Ma16B06 comprising a VHH sequence as shown in SEQ. ID NO: 15 or a VHH antibody, which is a variant thereof.

9. The VHH antibody of any one of claims 1-8, which is a monomeric VHH antibody.

10. The VHH antibody of any one of claims 1-8, which is a multimeric VHH antibody, e.g., a homotrimeric VHH antibody or a heterodimeric VHH antibody.

11. The VHH antibody of any one of claims 1-10, which is covalently or non-covalently conjugated to a heterologous moiety, e.g., a labeling group, a capture group or an effector group, wherein the heterologous moiety is particularly selected from a fluorescence group, biotin, an enzyme such as a peroxidase, phosphatase or luciferase, a hapten, an affinity tag, or a nucleic acid such as an oligonucleotide.

12. A set of two or more different VHH antibodies recognizing the SARS-CoV-2 spike protein S1 domain, particularly the receptor-binding domain (RBD) of the SARS-CoV-2 S1 domain, comprising at least one VHH antibody of any of claims 1-11, particularly a monomeric VHH antibody of claim 9.

13. The VHH antibody of any one of claims 1-11 or the set of claim 12 for use in medicine, e.g., human medicine, particularly for use in therapy or diagnostics.

14. The VHH antibody of any one of claims 1-11 or the set of claim 12 for use in the prevention or treatment of a disorder caused by and/or associated with an infection with SARS-CoV-2 including an infection with a SARS-CoV-2 escape mutant, particularly for use in the prevention or treatment of COVID-19 in a human subject.

15. A method for the prevention or treatment of a disorder caused by and/or associated with an infection with SARS-CoV-2, comprising administering an effective dose of the VHH antibody of any one of claims 1-11 or the set of claim 12 to a subject in need thereof, particularly to a human subject.
